# EUROPÄISCHE PATENTSCHRIFT

(11) **EP 0 303 878 B1**
(45) Veröffentlichungstag und Bekanntmachung des Hinweises auf die Patenterteilung: **13.07.1994**
(21) Anmeldenummer: 88112436.6
(22) Anmeldetag: 01.08.1988
(51) Int. Cl.: C07D 213/75, C07D 213/73, C07D 213/74, C07D 413/14, A61K 7/13

(54) **2,5-Diamino-6-nitro-pyridinderivate enthaltende Haarfärbemittel**
2,5-diamino-nitro-6-pyridine derivatives containing hair dyeing agents
Teintures pour cheveux a base des dérivés de diamino-2,5-nitro-6-pyridine

(30) Priorität: 17.08.1987 DE 3727297
(43) Veröffentlichungstag der Anmeldung: 22.02.1989
(60) Teilanmeldung: 93107242.5
(73) Patentinhaber: Wella Aktiengesellschaft, 64295 Darmstadt (DE)
(72) Erfinder: Clausen, Thomas, Dr., D-6146 Alsbach (DE); Konrad, Eugen, D-6100 Darmstadt (DE)

(56) Entgegenhaltungen:
- DE-A- 1 949 750
- DE-A- 3 334 030
- CHEMICAL ABSTRACTS, Band 95, 1981, Seite 675, Zusammenfassung Nr. 24817j, Columbus, Ohio, US; S.K. KOTOVSKAYA et al.: "2-nitro-3,6-diaminopyridines"
- CHEMICAL ABSTRACTS, Band 90, 1979, Seite 605, Zusammenfassung Nr. 152079d, Columbus, Ohio, US; G.A. MOKRUSHINA et al.: "Direct amination in the pyridine series"

## Beschreibung

Nitrofarbstoffe finden heute in Haarfärbemitteln eine breite Anwendung. Sie werden in Oxidationshaarfärbemitteln als Zusätze für die Erzeugung von natürlichen oder modischen Farbnuancen verwendet. Durch Kombination von mehreren verschieden gefärbten Nitrofarbstoffen ist es jedoch auch möglich, Haarfärbemittel herzustellen, die Haare in natürlichen bis modischen Nuancen ohne die Anwendung von Oxidationsmitteln zu färben vermögen.

So können zum Beispiel durch die Kombination je eines gelb-, rot- und blaufärbenden Nitrofarbstoffes natürlich wirkende braune Färbungen erzeugt werden. Daneben ist es jedoch zum Beispiel auch möglich, mit einem gelbfärbenden und einem violettfärbenden Nitrofarbstoff modische Brauntöne zu erhalten. Es werden daher Nitrofarbstoffe benötigt, die möglichst das gesamte Farbspektrum von gelb über orange, rot und violett bis blau abdecken. Zur Erzielung gleichmäßiger Färbungen an Ansatz und Spitzen der Haare sowie bei unterschiedlich geschädigten Haaren, ist es von Vorteil, wenn diese Farbstoffe aus einer Gruppe chemischer Verbindungen stammen, welche den gleichen Grundkörper aufweisen, weil dann leichter auch ähnliche physikalische Eigenschaften, die das Aufziehungsvermögen bestimmen, erzielbar sind.

Daneben werden noch viele weitere Anforderungen an Haarfarbstoffe gestellt.

Die Nitrofarbstoffe müssen in toxikologischer und dermatologischer Hinsicht unbedenklich sein. Ihre Verwendung in Oxidationshaarfärbemitteln setzt voraus, daß sie in Anwesenheit von Wasserstoffperoxid in alkalischer Lösung stabil sind. Außerdem wird für die erzeugten Haarfärbungen eine gute Licht-, Säure- und Reibechtheit gefordert. Schließlich sollten die Nitrofarbstoffe durch möglichst einfache, in industriellem Maßstab durchführbare Verfahren herstellbar sein.

In der Literatur (vergleiche zum Beispiel J. C. Johnson, Hair dyes, Noyes Data Corp., Park Ridge, New Jersey, USA 1973, Seiten 43-91 und Seiten 113-124) werden Nitrofarbstoffe ausführlich beschrieben. Gelbe, rote, violette und blaue Farbstoffe lassen sich bislang nur durch unterschiedlich substituierte 2-Nitro-p-phenylendiamine herstellen. Da nun in letzter Zeit Zweifel an der physiologischen Unbedenklichkeit mancher 2-Nitro-p-phenylendiaminderivate aufgekommen sind, besteht großer Bedarf an Farbstoffen mit einem nichtbenzoiden Grundkörper, welche bei möglichst gleicher Farb-Bandbreite bessere physiologische Eigenschaften aufweisen.

In der Literatur sind bereits Mittel zum Färben von Haaren mit einem Gehalt an Nitropyridinderivaten beschrieben. So werden in der DE-PS 1 949 750 Diaminopyridinderivate allgemein beschrieben. Als konkrete Beispiele werden jedoch nur 2,3-Diamino-5-nitro-pyridinderivate, welche menschliche Haare hellgelb bis orange färben, also nur ein begrenztes Farbspektrum aufweisen, genannt.

Weiterhin werden in der DE-OS 3 334 030 Mittel zur oxidativen Färbung von Haaren beschrieben, welche bestimmte Diaminonitropyridinderivate einer allgemeinen Formel enthalten sollen. In der Druckschrift werden jedoch keine konkreten Diaminonitropyridinderivate genannt. Da in der DE-OS 3 334 030 Nitropyridine grundsätzlich gemeinsam mit Entwicklungskomponenten eingesetzt werden und dort im Rahmen eines oxidativen Haarfarbsystems als Kupplungskomponenten reagieren sollen, erfolgt ihre Auswahl nach ihrer Kuppler-Eigenschaft.

Im Gegensatz hierzu können die in der vorliegenden Anmeldung beschriebenen 2,5-Diamino-6-nitro-pyridine zwar auch in Oxidationshaarfärbemitteln eingesetzt werden, sie reagieren jedoch nicht innerhalb des Systems, sondern werden als zusätzliche direktziehende Farbstoffe verwendet. Das Hauptanwendungsgebiet dieser Farbstoffe ist daher der Einsatz in direktziehenden Färbemitteln vom Typ der Tönung, welche keinen Zusatz von Oxidationsmitteln benötigen. Die hier vorliegenden 2,5-Diamino-6-nitro-pyridinderivate sind damit im Rahmen der DE-OS 3 334 030 überhaupt nicht verwendbar und weisen auch eine völlig andere chemische Struktur als die dort in den Beispielen beschriebenen Nitro-Kupplungskomponenten auf.

Die in der DE-OS 3 334 030 konkret beschriebenen Beispiele, welche unter anderem auch zu rötlichen Farbtönen führen, beruhen auf einer oxidativen Haarfärbung und sind im wesentlichen auf eine Eigenreaktion des dort eingesetzten 2,5-Diamino-pyridins zurückzuführen. Zudem sind die dort beschriebenen Nitroverbindungen in Gegenwart von Dithionit nicht reduktionsbeständig.

Somit ist aus der Literatur kein nichtbenzoides System von direkt auf das Haar aufziehenden Nitrofarbstoffen bekannt, welches die Haare von orange bis blauviolett zu färben vermag.

Es bestand deshalb die Aufgabe, ein Mittel zum Färben von Haaren auf der Grundlage eines Systems von direkt auf das Haar aufziehenden nichtbenzoiden Nitrofarbstoffen zur Verfügung zu stellen, welches intensive Haarfärbungen von gelborange bis blauviolett ermöglicht sowie in physiologischer Hinsicht unbedenklich ist.

Es wurde nun gefunden, daß durch ein Mittel zum Färben von Haaren mit einem Farbstoffgehalt und für Haarfärbemittel üblichen Zusätzen, dadurch gekennzeichnet, daß es ein 2,5-Diamino-6-nitro-pyridinderivat der allgemeinen Formel ( I) 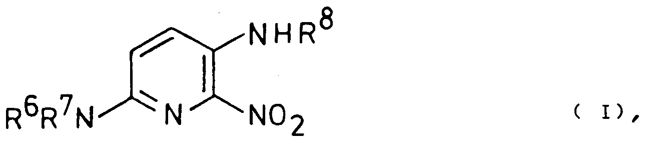
worin R⁶, R⁷ und R⁸ unabhängig voneinander Wasserstoff oder einen der Reste Alkyl, Hydroxyalkyl, Dihydroxyalkyl, Alkoxyalkyl, Aminoalkyl sowie mit Alkyl oder Hydroxyalkyl substituiertes Aminoalkyl bedeuten, wobei die Alkylreste jeweils 1 bis 4 Kohlenstoffatome aufweisen, oder durch R⁶ und R⁷ ein heterocyclischer, nicht aromatischer Fünf- oder Sechsring, der zusätzlich eine Oxygruppe aufweisen kann, gebildet wird, oder dessen physiologisch verträgliches wasserlösliches Salz enthält, die gestellte Aufgabe in hervorragender Weise gelöst wird.

Von den Verbindungen der Formel ( I) sind diejenigen bevorzugt, in denen R⁶, R⁷ und R⁸ unabhängig voneinander Wasserstoff oder einen der Reste Methyl, Ethyl oder 2-Hydroxyethyl bedeuten oder durch R⁶ und R⁷ ein Pyrrolidin-, Piperidin- oder Morpholinring gebildet wird.

Ganz besonders geeignete 2,5-Diamino-6-nitro-pyridinderivate sind 2,5-Diamino-6-nitro-pyridin, 5-Amino-2-((2'-hydroxyethyl)amino)-6-nitro-pyridin, 2,5-Bis((2'-hydroxyethyl)amino)-2-N-ethyl-6-nitro-pyridin, 2,5-Bis((2'-hydroxyethyl)amino)-6-nitro-pyridin, 2-Ethylamino-5-((2'-hydroxyethyl)amino)-6-nitro-pyridin und 5-Amino-2-pyrrolidino-6-nitro-pyridin.

Die Verbindungen der Formel ( I), welche teils aus der Literatur S. Kotovskaya et al., Khim. Geterotsikl. Soedin 1981, 654-657 (ref. CA 95, 114439s), G. A. Mokrushina et al., ibid. 1979, 131 (ref. CA 90, 152079d) und der russischen Patentschrift 773043 bekannt sind, teils weiter unten beschrieben sind, stellen für die Färbung von menschlichen Haaren hervorragend geeignete, je nach Variation der Substituenten R⁶ bis R⁷, gelborange bis blauviolett färbende Nitrofarbstoffe dar. Sie sind gut in Wasser löslich und weisen insbesondere als Bestandteil der erfindungsgemäßen Haarfärbemittel eine ausgezeichnete Lagerstabilität auf.

Überraschend sind die sehr guten toxikologischen und physiologischen Eigenschaften der Verbindungen der allgemeinen Formel ( I). So sind die getesteten Farbstoffe, zum Beispiel der Farbstoff mit R⁶ und R⁷ = 2-Hydroxyethyl und R⁸ = Wasserstoff, im Gegensatz zu strukturell ähnlichen Nitrofarbstoffen, wie zum Beispiel 1,4-Bis((2'-hydroxyethyl)amino)-2-nitro-benzol, im Amestest nicht mutagen.

Das erfindungsgemäße Mittel zur Färbung von Haaren betrifft sowohl eine Ausführungsform, bei der es ohne Zugabe eines Oxidationsmittels angewendet wird, als auch eine weitere Ausführungsform, bei der die Zugabe eines Oxidationsmittels erforderlich ist.

Bei dem ersteren Haarfärbemittel ohne Oxidationsmittel-Zugabe handelt es sich um ein Mittel, welches neben den Farbstoffen der angegebenen Formel ( I) noch andere bekannte direkt auf das Haar aufziehende Farbstoffe enthalten kann. Von diesen für die Haarfärbung bekannten Farbstoffen seien beispielsweise die folgenden erwähnt: Aromatische Nitrofarbstoffe, wie zum Beispiel 2-Amino-4-nitro-phenol, Pikraminsäure, 1-((2'-Hydroxyethyl)amino)-2-amino-4-nitro-benzol, 2-Nitro-4-((2'-hydroxyethyl)amino)-anilin, 4-((2',3'-Dihydroxypropyl)amino)-3-nitro-trifluormethylbenzol, 4-N,N'-Bis((2'-hydroxyethyl)amino)-1-methylamino-2-nitro-benzol, 2,5-Bis((2'-hydroxyethyl)amino)-nitrobenzol, 2-((2'-Hydroxyethyl)amino)-4,6-dinitro-phenol und 1-Amino-4-((2',3'-dihydroxypropyl)amino)-2-nitro-5-chlor-benzol; Triphenylmethanfarbstoffe, wie zum Beispiel Basic Violet 1 (C. I. 42 535); Azofarbstoffe, wie zum Beispiel Acid Brown 4 (C. I. 14 805); Anthrachinonfarbstoffe, wie zum Beispiel Disperse Blue 23 (C. I. 61 545), Disperse Violet 4 (C. I. 61 105), 1,4,5,8-Tetraamino-anthrachinon und 1,4-Diamino-anthrachinon, wobei die Farbstoffe dieser Klassen je nach Art ihrer Substituenten sauren, nichtionogenen oder basischen Charakter haben können. Weitere geeignete direkt auf das Haar aufziehende Farbstoffe sind beispielsweise in dem Buch von J.C. Johnson, "Hair dyes" Noyes Data Corp. Park-Ridge (USA) (1973), Seiten 3 - 91 und 113 - 139 (ISBN: 0-8155-0477-2) beschrieben.

Die Zubereitungsform des hier beschriebenen Haarfärbemittels auf der Basis von direkt auf das Haar aufziehenden Farbstoffen kann beispielsweise eine Lösung, insbesondere eine wäßrige oder wäßrig-alkoholische Lösung, sein. Bevorzugte Zubereitungsformen sind weiterhin eine Creme, ein Gel oder eine Emulsion, wobei das Mittel auch im Gemisch mit einem Treibgas oder mittels einer Pumpe versprüht werden kann.

Die Farbstoffe der allgemeinen Formel ( I) sollen in diesem Haarfärbemittel ohne Oxidationsmittel-Zugabe in einer Konzentration von etwa 0,01 bis 2,0 Gewichtsprozent, vorzugsweise von 0,01 bis 1,0 Gewichtsprozent, enthalten sein. Der Gesamtgehalt an den direkt auf das Haar aufziehenden Farbstoffen liegt in den Grenzen von etwa 0,01 bis 3,0 Gewichtsprozent.

Der pH-Wert dieses Färbemittels liegt im Bereich von 3 bis 10,5, insbesondere bei pH 7,5 bis 9,5, wobei die Einstellung des gewünschten alkalischen pH-Wertes hauptsächlich mit Ammoniak erfolgt, jedoch auch mit organischen Aminen, wie beispielsweise Monoethanolamin oder Triethanolamin, vorgenommen werden kann.

Seine Anwendung erfolgt in üblicher Weise durch Aufbringen einer für die Haarfärbung ausreichenden Menge des Mittels auf die Haare, mit denen es eine Zeitlang, etwa 5 bis 30 Minuten, in Berührung bleibt. Anschließend wird mit Wasser, gegebenenfalls noch mit der wäßrigen Lösung einer schwachen organischen Säure, gespült und getrocknet. Als schwache organische Säure können beispielsweise Essigsäure, Zitronensäure, Weinsäure und ähnliche Verwendung finden.

Das vorstehend beschriebene Haarfärbemittel ohne Oxidationsmittel-Zugabe kann selbstverständlich auch in der Kosmetik überlicherweise verwendete synthetische oder natürliche Polymere enthalten, wodurch gleichzeitig mit der Färbung eine Festigung der Haare erreicht wird. Solche Mittel werden im allgemeinen als Tönungsfestiger oder Farbfestiger bezeichnet. Von den für diesen Zweck in der Kosmetik bekannten synthetischen Polymeren seien beispielsweise Polyvinylpyrrolidon, Polyvinylacetat, Polyvinylalkohol oder Polyacrylverbindungen wie Acrylsäure- beziehungsweise Methacrylsäurepolymerisate, basische Polymerisate von Estern aus diesen beiden Säuren und Aminoalkoholen beziehungsweise deren Salze oder Quaternierungsprodukte, Polyacrylnitril, Polyvinyllactame sowie Copolymerisate aus derartigen Verbindungen, wie zum Beispiel Polyvinylpyrrolidon-Vinylacetat, und dergleichen erwähnt.

Auch natürliche Polymere, wie Chitosan (entacetyliertes Chitin) oder Chitosanderivate, können für den genannten Zweck eingesetzt werden.

Die Polymerisate sind in diesem Mittel in der für solche Mittel üblichen Menge von etwa 1 bis 5 Gewichtsprozent enthalten. Der pH-Wert des Mittels liegt im Bereich von etwa 6,0 bis 9,0.

Die Anwendung dieses Haarfärbemittels mit zusätzlicher Festigung erfolgt in bekannter und üblicher Weise durch Befeuchten des Haares mit dem Festiger, Festlegen (Einlegen) des Haares zur Frisur und anschließende Trocknung.

Selbstverständlich kann das vorstehend beschriebene Haarfärbemittel ohne Oxidationsmittel-Zugabe gegebenenfalls weitere, für Haarfärbemittel übliche Zusätze, wie zum Beispiel Pflegestoffe, Netzmittel, Verdicker, Weichmacher, Konservierungsstoffe und Parfümöle sowie auch andere, nachstehend für Oxidationshaarfärbemittel aufgeführte übliche Zusätze enthalten.

Zum Gegenstand der vorliegenden Erfindung gehört auch, wie eingangs erwähnt, ein Haarfärbemittel, bei dem die Zugabe eines Oxidationsmittels erforderlich ist. Es enthält außer den Farbstoffen gemäß der angegebenen Formel ( I) und gegebenenfalls bekannten, direkt auf das Haar aufziehenden Farbstoffen noch zusätzliche, bekannte Oxidationsfarbstoffe, die einer oxidativen Entwicklung bedürfen.

Bei diesen Oxidationsfarbstoffen handelt es sich hauptsächlich um aromatische p-Diamine und p-Aminophenole, wie beispielsweise p-Toluylendiamin, p-Phenylendiamin, p-Aminophenol und ähnliche Verbindungen, welche zum Zwecke der Nuancierung der Färbung mit sogenannten Modifiern, wie zum Beispiel m-Phenylendiamin, Resorcin, m-Aminophenol und anderen, kombiniert werden.

Derartige zu Haarfärbung bekannte und übliche Oxidationsfarbstoffe sind unter anderem in dem Buch von E. Sagarin, "Cosmetics", Science and Technology (1957), Interscience Publishers Ins., New York, Seiten 503 ff. sowie in dem Buch von H. Janistyn, "Handbuch der Kosmetika und Riechstoffe" (1973), Seiten 338 ff., beschrieben.

Mit Mischungen aus diesen Oxidationsfarbstoffen und den Farbstoffen gemäß der angegebenen Formel ( I) lassen sich sehr gut natürliche Blond- und Brauntöne, aber auch modische Nuancen herstellen.

Die Farbstoffe gemäß der Formel ( I) sind in diesem Färbemittel mit Oxidationsmittel-Zugabe in einer Konzentration von etwa 0,01 bis 2,0 Gewichtsprozent, vorzugsweise 0,01 bis 1,0 Gewichtsprozent, enthalten. Der Gesamtgehalt an Farbstoffen in diesem Färbemittel beträgt etwa 0,1 bis 5,0 Gewichtsprozent.

Oxidationshaarfärbemittel sind im allgemeinen alkalisch eingestellt, vorzugsweise auf pH-Werte von 8,0 bis 11,5, wobei die Einstellung insbesondere mit Ammoniak erfolgt. Es können dazu aber auch andere organische Amine, zum Beispiel Monoethanolamin oder Triethanolamin, Verwendung finden. Als Oxidationsmittel zur Entwicklung der Haarfärbungen kommen hauptsächlich Hydrogenperoxid und dessen Additionsverbindungen in Betracht. Die Zubereitungsform dieses Haarfärbemittels kann die gleiche wie bei dem Haarfärbemittel ohne Oxidationsmittel-Zugabe sein. Vorzugsweise ist sie die einer Creme oder eines Gels.

Übliche Zusätze in Cremes, Emulsionen oder Gelen sind zum Beispiel Lösungsmittel, wie Wasser, niedere aliphatische Alkohole, beispielsweise Ethanol, Propanol, Isopropanol, Glycerin oder Glykole, wie zum Beispiel Ethylenglykol und Propylenglykol, oder auch Glykolether, weiterhin Netzmittel oder Emulgatoren aus den Klassen der anionischen, kationischen, amphoteren oder nichtionogenen oberflächenaktiven Substanzen, wie zum Beispiel Fettalkoholsulfate, Fettalkoholethersulfate, Alkylsulfonate, Alkylbenzolsulfate, Alkyltrimethylammoniumsalze, Alkylbetaine, oxethylierte Fettalkohole, oxethylierte Alkylphenole, Fettsäurealkanolamide, oxethylierte Fettsäureester, ferner Verdicker, wie zum Beispiel höhere Fettalkohole, Bentonit, Stärke, Polyacrylsäure, Cellulosederivate, wie zum Beispiel Carboxymethylcellulose, Alginate, Vaseline, Paraffinöl und Fettsäuren sowie außerdem Pflegestoffe, wie zum Beispiel Lanolinderivate, Cholesterin, Pantothensäure und Betain, weiterhin Parfümöle und Komplexbildner. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, während die Verdicker in einer Menge von etwa 0,1 bis 25 Gewichtsprozent und die Pflegestoffe in einer Menge von etwa 0,1 bis 5 Gewichtsprozent in den Zubereitungen enthalten sein können.

Die Anwendung der genannten Zubereitungen, bei denen die Zugabe eines Oxidationsmittels erforderlich ist, erfolgt in bekannter Weise, indem man die Haarfärbemittel vor der Behandlung mit dem Oxidationsmittel vermischt und eine zur Färbung des Haares ausreichende Menge der Mischung, im allgemeinen etwa 50 bis 150 ml, auf das Haar aufträgt. Mach einer für die Haarfärbung ausreichenden Einwirkungszeit, welche üblicherweise etwa 10 bis 45 Minuten beträgt, wird das Haar mit Wasser gespült, gegebenenfalls anschließend mit der wäßrigen Lösung einer schwachen organischen Säure, wie zum Beispiel Zitronensäure oder Weinsäure, nachgespült und sodann getrocknet.

Hinsichtlich der färberischen Möglichkeiten bieten die erfindungsgemäßen Haarfärbemittel je nach Art und Zusammensetzung der Farbkomponenten eine breite Palette verschiedener Farbnuancen, die sich von natürlichen Farbtönen bis hin zu hochmodischen, leuchtenden Nuancen erstreckt. Hierbei werden die Färbemittel je nach Zusammensetzung entweder in Verbindung mit Hydrogenperoxid oder auch ohne Oxidationsmittel angewandt.

Die Verbindungen der Formel (I) lassen sich nach dem in der EP-OS 0 560 406 (welche eine Ausscheidungsanmeldung zur vorliegenden Anmeldung ist) beschriebenen Verfahren in guten Ausbeuten herstellen.

Die nachfolgenden Beispiele sollen den Gegenstand der Erfindung näher erläutern.

### Beispiele für Haarfärbemittel

### Beispiel 1 flüssiges Haarfärbemittel

| | |
|---|---|
| 0,50 g | 2,5-Diamino-6-nitro-pyridin |
| 2,00 g | Laurylalkoholdiglykolethersulfat-Natriumsalz (28prozentige wäßrige Lösung) |
| 2,00 g | Ammoniak, 25prozentige wäßrige Lösung |
| 95,50 g | Wasser |
| 1̅0̅0̅,̅0̅0̅ g̅ | |

Gebleichtes Naturhaar wird 20 Minuten lang bei Raumtemperatur mit der Lösung nach Beispiel 1 behandelt, danach mit Wasser ausgespült und getrocknet. Das Haar ist goldorange gefärbt.

### Beispiel 2 Farbfestiger

| | |
|---|---|
| 0,10 g | 2-Ethylamino-5-((2'-hydroxyethyl)amino)-6-nitro-pyridin |
| 2,00 g | Polyvinylpyrrolidon |
| 0,10 g | Glyzerin |
| 40,00 g | Isopropanol |
| 57,80 g | Wasser |
| 1̅0̅0̅,̅0̅0̅ g̅ | |

Weiße menschliche Haare werden mit der Farbfestiger-Lösung eingelegt und getrocknet. Das Haar ist leuchtend rotviolett gefärbt und gefestigt.

### Beispiel 3 Oxidationshaarfärbemittel in Cremeform

| | |
|---|---|
| 0,20 g | 2,5-Bis-((2'-hydroxyethyl)amino)-6-nitro-pyridin |
| 0,02 g | 4-((2'-Hydroxyethyl)amino)-3-nitro-toluol |
| 0,20 g | p-Phenylendiamin |
| 0,15 g | Resorcin |
| 0,03 g | m-Aminophenol |
| 15,00 g | Cetylalkohol |
| 3,50 g | Laurylalkoholdiglykolethersulfat-Natriumsalz (28prozentige wäßrige Lösung) |
| 6,00 g | Ammoniak, 25prozentige wäßrige Lösung |
| 74,90 g | Wasser |
| 1̅0̅0̅,̅0̅0̅ g̅ | |

50 g des vorstehenden Haarfärbemittels werden kurz vor der Anwendung mit 50 ml Wasserstoffperoxidlösung (6prozentig) gemischt. Das Gemisch wird anschließend auf graue menschliche Haare aufgetragen und 30 Minuten bei einer Temperatur von 40 Grad Celsius einwirken gelassen. Nach dem Spülen des Haares mit Wasser und dem anschließenden Trocknen hat es einen modischen roten Blondton angenommen.

### Beispiel 4 Haarfärbemittel in Cremeform

| | |
|---|---|
| 0,030 g | 5-Amino-2-pyrrolidino-6-nitro-pyridin |
| 0,050 g | 4-((2',3'-Dihydroxypropyl)amino)-3-nitro-trifluormethylbenzol |
| 0,250 g | 4-N,N'-Bis((2'-hydroxyethyl)amino)-1-methylamino-2-nitro-benzol |
| 0,025 g | Disperse Blue 23 (C. I. 61 545) |
| 7,500 g | Cetylalkohol |
| 1,750 g | Lauryalkoholdiglykolethersulfat-Natriumsalz (28prozentige wäßrige Lösung) |
| 0,100 g | p-Hydroxybenzoesäuremethylester |
| 0,200 g | Ammoniak, 25prozentige wäßrige Lösung |
| 90,095 g | Wasser |
| 1̅0̅0̅,̅0̅0̅ g̅ | |

50 g des vorstehenden Haarfärbemittels werden auf weiße menschliche Haare aufgebracht und nach einer Einwirkungszeit von 20 Minuten mit Wasser ausgespült. Das Haar wird anschließend getrocknet. Es ist in einem natürlichen braunen Farbton gefärbt.

### Beispiel 5 Flüssiges Haarfärbemittel

| | |
|---|---|
| 0,10 g | 2,5-Bis((2'-hydroxyethyl)amino)-2-N-ethyl-6-nitro-pyridin |
| 0,25 g | 4-((2'-Hydroxyethyl)amino)-3-nitro-chlorbenzol |
| 0,20 g | 4-N-Ethyl-1,4-bis((2'-hydroxyethyl)amino)-2-nitro-benzol |
| 0,50 g | Hydroxyethylcellulose |
| 5,00 g | Lauryalkoholdiglykolethersulfat-Natriumsalz (28prozentige wäßrige Lösung) |
| 10,00 g | Isopropanol |
| 10,00 g | Ammoniak, 25prozentige wäßrige Lösung |
| 73,95 g | Wasser |
| 1̅0̅0̅,̅0̅0̅ g̅ | |

Gebleichtes Naturhaar wird 20 Minuten lang bei Raumtemperatur mit der Lösung nach Beispiel 5 behandelt. Nach Spülung mit Wasser und Trocknung ist das Haar in einem modischen dunklen Beaujolaiston eingefärbt.

### Beispiel 6 flüssiges Haarfärbemittel

| | |
|---|---|
| 0,05 g | 2,5-Bis((2'-hydroxyethyl)amino)-2-N-ethyl-6-nitro-pyridin |
| 0,30 g | 4-((2'-Ureidoethyl)amino)-nitrobenzol |
| 5,00 g | Laurylalkoholdiglykolethersulfat-Natriumsalz (28prozentige wäßrige Lösung) |
| 10,00 g | Isopropanol |
| 10,00 g | Ammoniak, 25prozentige wäßrige Lösung |
| 74,65 g | Wasser |
| 1̅0̅0̅,̅0̅0̅ g̅ | |

Das vorstehende Färbemittel wird auf gebleichte Naturhaare 30 Minuten lang bei 40 Grad Celsius einwirken gelassen. Nach Spülung mit Wasser und Trocknung ist das Haar in einem modischen Blondton gefärbt.

### Beispiel 7 Haarfärbemittel in Cremeform

| | |
|---|---|
| 0,7 g | 5-Amino-2-((2'-hydroxyethyl)amino)-6-nitro-pyridin |
| 10,0 g | Cetylstearylalkohol |
| 5,0 g | Laurylalkoholdiglykolethersulfat-Natriumsalz (28prozentige wäßrige Lösung) |
| 0,5 g | Parfüm |
| 0,1 g | Natriumhydroxid |
| 83,7 g | Wasser |
| 1̅0̅0̅,̅0̅ g̅ | |

Das Färbemittel läßt man 20 Minuten auf blonde Naturhaare bei 20 Grad Celsius einwirken. Nach Spülung und Trocknung ist das Haar leuchtend orange eingefärbt.

### Beispiel 8 Haarfärbemittel in Cremeform

Wird im Beispiel 7 das 5-Amino-2-((2'-hydroxyethyl)amino)-6-nitro-pyridin durch 2,5-Bis((2'-hydroxyethyl)amino)-6-nitro-pyridin in gleicher Menge ersetzt, so wird das Haar leuchtend feuerrot gefärbt.

Die in dieser Anmeldung verwendeten Prozentzahlen stellen, soweit nicht anders angegeben, Gewichtsprozente dar.

## Patentansprüche

1. Mittel zum Färben von Haaren mit einem Farbstoffgehalt und für Haarfarbemittel üblichen Zusätzen, dadurch gekennzeichnet, daß es ein 2,5-Diamino-6-nitro-pyridinderivat der allgemeinen Formel ( I) 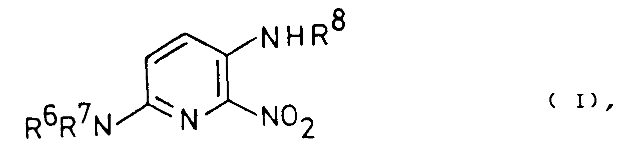 worin R⁶, R⁷ und R⁸ unabhängig voneinander Wasserstoff oder einen der Reste Alkyl, Hydroxyalkyl, Dihydroxyalkyl, Alkoxyalkyl, Aminoalkyl, sowie mit Alkyl oder Hydroxyalkyl substituiertes Aminoalkyl bedeuten, wobei die Alkylreste jeweils 1 bis 4 Kohlenstoffatome aufweisen, oder durch R⁶ und R⁷ ein heterocyclischer, nicht aromatischer Fünf- oder Sechsring, der zusätzlich eine Oxygruppe aufweisen kann, gebildet wird, oder dessen physiologisch verträgliches wasserlösliches Salz enthält.

2. Mittel nach Anspruch 1, dadurch gekennzeichnet, daß es ein 2,5-Diamino-6-nitro-pyridinderivat der Formel ( I) enthält, worin R⁶, R⁷ und R⁸ unabhängig voneinander Wasserstoff, Methyl, Ethyl oder 2-Hydroxyethyl bedeuten oder durch R⁶ und R⁷ ein Pyrrolidin-, Piperidin- oder Morpholinring gebildet wird.

3. Mittel nach einem der Ansprüche 1 oder 2, dadurch gekennzeichnet, daß das 2,5-Diamino-6-nitro-pyridinderivat der Formel ( I) ausgewählt ist aus 2,5-Diamino-6-nitro-pyridin, 5-Amino-2-((2'-hydroxyethyl)amino)-6-nitro-pyridin, 2,5-Bis((2'-hydroxyethyl)amino)-2-N-ethyl-6-nitro-pyridin, 2,5-Bis((2'-hydroxyethyl)-amino)-6-nitro-pyridin, 2-Ethylamino-5-((2'-hydroxyethyl)amino)-6-nitro-pyridin und 5-Amino-2-pyrrolidino-6-nitro-pyridin.

4. Mittel nach einem der Ansprüche 1 bis 3 , dadurch gekennzeichnet, daß das 2,5-Diamino-6-nitro-pyridinderivat der Formel ( I) in einer Menge von 0,01 bis 2,0 Gewichtsprozent enthalten ist.

5. Mittel nach einem der Ansprüche 1 bis 4, dadurch gekennzeichnet, daß es mindestens einen der bekannten, direkt auf das Haar aufziehenden Farbstoffe enthalt.

6. Mittel nach Anspruch 5, dadurch gekennzeichnet, daß die bekannten, direkt auf das Haar aufziehenden Farbstoffe ausgewählt sind aus 2-Amino-4-nitrophenol, Pikraminsäure, 1-((2'-Hydroxyethyl)amino)-2-amino-4-nitro-benzol, 2-Nitro-4-((2'-hydroxyethyl)amino)-anilin, 4-((2',3'-Dihydroxypropyl)amino)-3-nitro-trifluormethylbenzol, 4-N,N'-Bis((2'-Hydroxyethyl)amino)-1-methylamino-2-nitrobenzol, 2,5-Bis((2'-hydroxyethyl)amino)-nitrobenzol, 2-((2'-Hydroxyethyl)- amino)-4,6-dinitro-phenol, 1-Amino-4-((2',3'-dihydroxypropyl)amino)-2-nitro-5-chlor-benzol, Basic Violet 1 (C.I.42 535), Acid Brown 4 (C.I.14 805), Disperse Violet 4 (C.I. 61 105), Disperse Blue 23 (C.I.61 545), 1,4,5,8-Tetraamino-anthrachinon und 1,4-Diamino-anthrachinon.

7. Mittel nach einem der Ansprüche 5 oder 6, dadurch gekennzeichnet, daß es in Form eines Haarfärbemittels mit zusätzlicher Haarfestigung vorliegt und mindestens ein in der Kosmetik üblicherweise verwendetes synthetisches oder natürliches Polymer enthält.

8. Mittel nach Anspruch 7, dadurch gekennzeichnet, daß das synthetische oder natürliche Polymer ausgewählt ist aus Chitosan und dessen Derivaten, Polyvinylpyrrolidon, Polyvinylacetat, Polyvinylalkohol, Polyacrylsäure, Polymethacrylsäure, Polyacrylnitril, Polyvinyllactam oder Copolymerisaten aus diesen Verbindungen.

9. Mittel nach einem der Ansprüche 1 bis 4, dadurch gekennzeichnet, daß es mindestens einen der bekannten Oxidationshaarfarbstoffe enthält.

10. Mittel nach Anspruch 9, dadurch gekennzeichnet, daß der bekannte Oxidationshaarfarbstoff ausgewählt ist aus p-Toluylendiamin, p-Phenylendiamin, p-Aminophenol, m-Phenylendiamin, Resorcin und m-Aminophenol.

## Claims

1. Medium for colouring hair with a dye content and conventional additives for hair colouring media, characterised in that it contains a 2,5 - diamino-6-nitro-pyridine derivative of the general formula ( I) 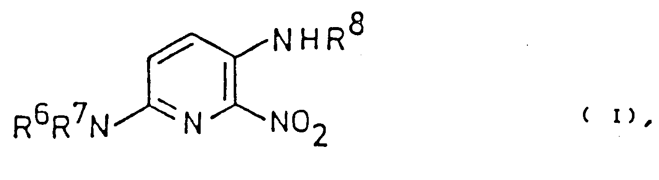 wherein R⁶ , R⁷ and R⁸, independently of each other mean hydrogen or one of the residues alkyl, hydroxyalkyl, dihydroxyalkyl, alkoxyalkyl, aminoalkyl and also aminoalkyl substituted with alkyl or hydroxyalkyl, wherein the alkyl residues have 1 to 4 carbon atoms in each case, or a heterocyclic non-aromatic, five-or six-membered ring, which in addition can have an oxygroup, is formed by R⁶ and R⁷, or a physiologically tolerable water-soluble salt thereof.

2. Medium according to Claim 1, characterised in that it contains a 2,5-diamino-6-nitro-pyridine derivative of the formula ( I), wherein R⁶ , R⁷ and R⁸ , independently of each other mean hydrogen, methyl, ethyl or 2-hydroxyethyl, or a pyrrolidine,piperidine, or morpholine ring is formed by R⁶ and R⁷ .

3. Medium according to either Claim 1 or 2, characterised in that the 2,5 - diamino-6-nitro-pyridine derivative of the formula ( I) is selected from 2,5-diamino-6-nitro-pyridine, 5-amino-2-((2'-hydroxyethyl)amino)-6-nitro-pyridine, 2,5-bis((2'-hydroxy ethyl)amino)-2-N-ethyl-6-nitro-pyridine, 2,5-bis((2'-hydroxyethyl)-amino)-6-nitro-pyridine, 2-ethyl amino-5-((2'-hydroxyethyl)amino)-6-nitro-pyridine and 5-amino-2-pyrrolidine-6-nitro-pyridine.

4. Medium according to any one of the Claims 1 to 3, characterised in that the 2,5-diamino-6-nitro-pyridine derivative of the formula ( I) is contained in a quantity of 0.01 to 2.0 weight %.

5. Medium according to any one of the Claims 1 to 4, characterised in that it contains at least one of the known dyes which is absorbed directly onto the hair.

6. Medium according to Claim 5, characterised in that the known dyes which are absorbed directly onto the hair are selected from 2-amino-4 nitro phenol, picramic acid, 1-((2'-hydroxyethyl)-amino)-2-amino-4-nitro-benzene, 2-nitro-4-((2'-hydroxyethyl)amino)-analine, 4-((2',3'-dihydroxy propyl)amino)-3-nitro-trifluoromethylbenzene, 4-N,N' bis ((2'-hydroxyethyl)amino)-1-methylamino-2-nitrobenzene, 2,5-bis((2'-hydroxyethyl)amino) nitrobenzene, 2 ((2'-hydroxyethyl)-amino)-4,6-dinitrophenol,1-amino-4-((2'3'-dihydroxypropyl)amino)-2-nitro-5-chlorbenzene, Basic Violet 1 (C.I. 42 535), Acid Brown 4 (C I. 14 805), Disperse Violet 4 (C.I. 61 105), Disperse Blue 23 (C.I. 61 545), 1,4,5,8-tetra amino anthraquinone and 1,4 - diamino-anthraquinone.

7. Medium according to either of Claims 5 or 6, characterised in that it is in the form of a hair colouring medium with additional hair strengthener and contains at least one synthetic or natural polymer conventionally used in cosmetics.

8. Medium according to Claim 7, characterised in that the synthetic or natural polymer is selected from chitosan or its derivatives, polyvinylpyrrolidone, polyvinylacetate, polyvinylalcohol, polyacrylic acid, polymethacrylic acid , polyacrylonitrile, polyvinyl lactam or copolymers of these compounds.

9. Medium according to any one of the Claims 1 to 4, characterised in that it contains at least one of the known oxidation hair dyes.

10. Medium according to Claim 9, characterised in that the known oxidation hair dye is selected from p-toluene diamine, p-phenylene diamine, p-amino phenol, m-phenylene diamine, resorcinol and m-aminophenol.

## Revendications

1. Produit de coloration des cheveux contenant des colorants et des additifs usuels pour les produits de coloration, caractérisé en ce qu'il renferme un dérive de la 2,5-diamino-6-nitro-pyridine répondant à la formule générale ( I). 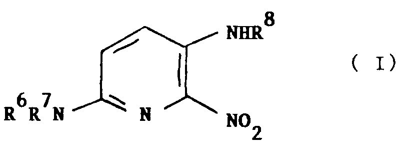 Dans laquelle R⁶, R⁷ et R⁸ représentent, indépendamment les uns des autres, l'hydrogène ou l'un des restes alkyle, hydroxyalkyle, dihydroxyalkyle, alkoxyalkyle, aminoalkyle, et aminoalkyle substitué par un alkyle ou un hydroxyalkyle, les restes alkyle comportant chacun 1 à 4 atomes de carbone, ou bien R⁶ et R⁷ formant un cercle hétérocyclique pentagonal ou hexagonal non-aromatique qui peut comporter en plus un groupe oxy, ou son sel soluble dans l'eau physiologiquement neutre.

2. Produit selon la revendication 1, caractérisé en ce qu'il renferme un dérivé de la 2,5-diamino-6-nitro pyridine répondant à la formule génrale ( I), dans laquelle R⁶, R⁷et R⁸ représentent, indépendamment les uns des autres, l'hydrogène ou un groupe méthyle, éthyle ou 2-hydroxyéthyle, ou bien R⁶ et R⁷ forment un cercle pyrrolidinique, pipéridinique ou morpholinique.

3. Produit selon la revendication 1 ou 2, caractérisé en ce que le dérivé 2,5-diamino-6-nitropyridinique répondant à la formule ( I) est choisi parmi la 2,5-diamino-6-nitro-pyridine, la 5-amino-2-((2'-hydroxyéthyl)-amino)-6-nitro-pyridine, la 2,5-bis(2'-hydroxyéthyl)amino)-2-N-éthyl-6-nitro-pyridine, la 2,5-bis ((2'-hydroxyéthyl)amino)-6-nitro-pyridine, la 2-éthylamino-5-((2'-hydroxyéthyl)amino)-6-nitro-pyridine et la 5-amino-2-pyrrolidino-6-nitro-pyridine.

4. Produit selon l'une des renvendications 1 à 3, caractérisé en ce que le dérivé de le 2,5-diamino-6-nitropyridine répondant à la formule (I) est présent dans une production de 0̸,0̸1 à 2,0̸% en poids,

5. Produit seton l'une des revendications 1 à 4, caractérisé en ce qu'il renferme l'un au moins des colorants prenant directement sur les cheveux connus.

6. Produit selon la revendication 5, caractérisé en ce que les colorants prenant directement sur les cheveux connus sont choisis parmi le 2-amino-4-nitrophénol, l'acide picramique, le 1-((2'-hydroxyéthyl)-amino)-2-amino-4-nitrobenzène, la 2-nitro-4-((2'-hydroxyéthyl)amino)-aniline, le 4-(2', 3'-dihydroxypropyl)amino)-3-nitro-trifluorométhyl-benzène, le 4-N,N' bis((2'-hydroxyéthyl)amino)-1-méthylamino-2-nitrobenzène, le 2,5-bis-((2'-hydroxyéthyl)amino)nitrobenzène, le 2-((2'-hydroxyéthyl)-amino)-4,6-dinitro phénol, le 1-amino-4-((2', 3'-dihydroxypropyl)amino)-2-nitro-5-chlorobenzène, Basic Violet 1 (I.C. 42 535), Acid Brown 4 (I.C. 14 80̸5), Disperse Violet 4 (I.C. 61 10̸5), Disperse Blue 23 (I.C. 61 545), la 1,4,5,8-tétraamino-anthraquinone et la 1,4-diamino-anthraquinone.

7. Produit selon la revendication 5 ou 6, caractérisé en ce qu'il se présente sous le forme d'un colorant capillaire à fixation des cheveux supplémentaires et renferme au moins un polymère naturel ou synthétique utilisé couramment en cosmétique.

8. Produit selon la revendication 7, caractérisé en ce que le polymère naturel ou synthétique est choisi parmi le chitosane et ses dérivés, la polyvinylpyrrolidone, l'acétate de polyvinyle, l'acool polyvinylique, l'acide polyacrylique, l'acide polyméthacrylique, le polyacrylonitrille, le polyvinyl-lactame ou des copolymérisats de ces composés.

9. Produit selon l'une des revendications 1 à 4, caractérisé en ce qu'il renferme l'un au moins des colorants d'oxydation capillaires connus.

10. Produit selon la revendication 9, caractérisé en ce que le colorant d'oxydation connu est choisi parmi la p-toluèlène-diamine, la p-phénylène-diamine, le p-aminophénol, la m-phénylène-diamine, la résorcine et le m-aminophénol.
